# EUROPEAN PATENT APPLICATION

(11) **EP 1 759 718 A1**
(43) Date of publication of application: **07.03.2007**
(21) Application number: 05741543.2
(22) Date of filing: 18.05.2005
(51) Int. Cl.: A61L 24/00, A61K 9/70, A61K 33/06, A61K 38/43, A61K 38/48, A61K 45/00, A61L 15/64, A61L 17/00, A61L 27/00, A61P 7/04

(54) **TISSUE CLOSING PREPARATION**

(30) Priority: 21.05.2004 JP 2004152474
(71) Applicant: Juridical Foundation, The Chemo-Sero-Therapeutic Research Institute, Kumamoto-shi, Kumamoto 860-8568 (JP)
(72) Inventor: IMAMURA, Takayuki, Chemo-Sero-Therapeutic Res Inst, Kikuchi-shi, Kumamoto 8691298 (JP); SHINYA, Noriko The Chemo-Sero-Therapeutic Res Inst, Kikuchi-shi, Kumamoto 8691298 (JP); KAWAMURA, Ryoichi, Chemo-Sero-Therapeutic Res Inst, Kikuchi-shi, Kumamoto 8691298 (JP); NOZAKI, Chikateru, Chemo-Sero-Therapeutic Res Inst, Kikuchi-shi, Kumamoto 8691298 (JP)
(74) Representative: Vossius & Partner
(86) International application number: PCT/JP2005/009065
(87) International publication number: WO 2005/113030

(57) **Abstract**

The present invention relates to a tissue sealant which is safe and effective. In accordance with the present invention, a tissue sealant comprising as an effective ingredient thrombin and fibrinogen **characterized in that** a bioabsorbable synthetic nonwoven fabric is used as a supporting material; use of a combination of a bioabsorbable synthetic nonwoven fabric as a supporting material and thrombin and fibrinogen as an effective ingredient for a tissue sealant; use of a combination of a bioabsorbable synthetic nonwoven fabric holding thrombin as an effective ingredient, and fibrinogen as an effective ingredient for a tissue sealant; a tissue sealing kit comprising a bioabsorbable synthetic nonwoven fabric holding thrombin as an effective ingredient, and a container comprising fibrinogen as an effective ingredient; and a tissue sealing kit comprising a bioabsorbable synthetic nonwoven fabric as a substrate, a container comprising thrombin as an effective ingredient and a container comprising fibrinogen as an effective ingredient are provided.

## Description

### TECHNICAL FIELD OF THE INVENTION

The present invention relates to a tissue sealant comprising as an effective ingredient thrombin and fibrinogen characterized in that a bioabsorbable synthetic nonwoven fabric is used as a supporting material.

### BACKGROUND OF THE INVENTION

In various surgical operations, a tissue sealing is a treatment required in many cases. For instance, a tissue sealing is required for a defect site or an incised surface of the organs and tissues or junction between incised tissues or between incised tissues and prosthetic materials.

By way of example, in case of a defect site of membranous tissues, the heart in case of the pericardium or the intestinal tract in case of the peritoneum may possibly be caused to induce adhesion with the surrounding tissues or strangulation through the defect site to thereby incur malfunction. Thus, sealing of the defect site with prosthetic materials is sometimes carried out. However, it is not infrequently reported that detrimental events such as adhesion or chronic inflammation or infection are caused by these prosthetic materials.

For sealing of a defect site or an incised surface of the organ and tissues, e.g. in case of the organs such as the lung or the digestive organ, incomplete sealing would be accompanied by a risk of fatality due to malfunction or, even if evading a risk of fatality, would result in considerable decline in QOL. For a tissue sealing, suture or a combination of suture and a supporting material is sometimes selected. However, in case of fragile tissues, it is not infrequent that the tissue tears upon sticking with a needle and, suture and suture again, the tissue keeps tearing to prolong an operation time. Moreover, for such fragile tissues, even if a tissue sealing is completed, its effect may be insufficient and relapse may often occur and hence a patient will be compelled to undergo another surgical operation. Such a problem may also be seen in a surgical operation of the blood vessel for sealing junction between incised tissues or between incised tissues and prosthetic materials.

To ensure an effective tissue sealing, a supporting material or a fibrin sealant may often be used together with a suture but there is room for further improvement. The conventional tissue sealing approaches as well as the problems to be solved by the present invention are hereinbelow explained in detail taking as an example the field of cardiovascular surgery and respiratory surgery.

One of the problems to be solved in relation to a tissue sealing in the field of cardiovascular surgery is sealing of the pericardium after surgical operation of the heart. Defect in the pericardium may often occur after surgical operation of the heart and how a defect site in the pericardium is sealed may seriously affect especially when further operation is performed.

In recent years, an open heart operation has been carried out even in juvenility and in complicated cases with improvement of performance wherein divided operations by design are routinely conducted as an option of therapy. Accordingly, a further operation may be required in increasing cases especially for infants. Adhesion between the heart and its surrounding tissues such as the pericardium and the sternum and also cicatrization in the epicardium become problems in subsequent operations. When adhesion occurs, not only much time will be required for the separation and hemorrhage of the adhered tissues but also possibility to injure the heart and the great vessels while separation as well as lethality will increase. In case that cicatrization in the epicardium occurs from adhesion or chronic pericarditis, the tract of the coronary artery may not be observed with the eye to render a further operation of the coronary artery difficult. Developing into constrictive endocarditis, not confined to the matter of a further operation in this case, even heart failure due to impairment in cardiac dilation will be demonstrated.

Main causes of postoperative adhesion or cicatrization in the epicardium as described above appear to be a treatment of the pericardium while a surgical operation. In case that a graft or the heart is pressed by suture of the pericardium such as e.g. in case of a coronary artery bypass operation or an operation using extra-cardiac conduit, the pericardium may not occasionally be sealed. However, if the pericardium is not sealed, adhesion may occur to thereby increase a risk of fatality while a further operation and hence, in most cases of high possibility of a further operation, a defect site of the pericardium is filled with a sheet made of EPTFE. However, even if a sheet made of EPTFE is used, adhesion or cicatrization in the epicardium may still be observed in some cases and besides the sheet is problematic in that it may become the focus of infection due to its non-absorbability and that it may not fit for growth of a patient. Accordingly, there is a need for a sealing material that has good biocompatibility, does not induce excessive adhesion or inflammation, and is gradually degraded and absorbed in the living body while being replaced with the living tissue to effectively maintain sealing of the pericardium.

A tissue sealing in the field of respiratory surgery is typically a sealing of an air leakage from a peeled surface of the visceral pleura, an incised surface of the lung or the stump of the bronchia. Clinically, a sealing of an air leakage is done by applying a fibrin sealant after suture with a fibrin sealant alone or in combination with a supporting material. However, relapse of an air leakage may frequently be observed after an operation and in some institutions the relapse is found in the ratio of eight to ten surgical patients. Postoperative relapse of an air leakage may prevent early removal of a drain or early leaving from the hospital and also may lead to postoperative complications such as empyema. An extreme air leakage may sometimes exacerbate respiratory conditions to necessitate a further operation. Moreover, when bronchorrhea occurs after excision of the lobes of lung or extirpation of the whole lung, a further operation is impossible and it may often be untreatable. In recent years, with the increasing number of lung cancer cases with complication of emphysema in the aged, a postoperative relapse of an air leakage has become a grave concern. A possible cause of this is thought to be an insufficient tissue sealing while an operation. Therefore, a more effective means for sealing is desired that has thorough pressure resistance and may preclude an air leakage.

In recent years, segmental excision has increasingly been performed for a small-size lung cancer or atypical mycobacteriosis and hence a sealing approach has become desired that does not need to suture an incised surface of a segment to maintain the shape of the remaining lung and to reserve the pulmonary function. Its necessity becomes pressing with the increasing number of cases accompanied by emphysema wherein suture is difficult due to fragility of the tissues.

A fibrin sealant is used for a tissue adhesion, sealing and hemostasis of tissues by overlaying fibrinogen and thrombin solutions on wounded regions or by applying a mixed solution of fibrinogen and thrombin with a spraying device. However; when a fibrin sealant is applied by overlaying, both the fibrinogen and thrombin solutions would often flow away, in particular, at a surface of a high slope. Besides, a formed fibrin gel would become inhomogeneous. On the other hand, when the mixed solution is applied by spraying, it is reported that the solution would not likely to flow away and a formed fibrin gel is homogeneous but the solution apt to coagulate before penetrating deep into the interior of the tissues. Therefore, though depending on what the tissue is, a sufficient sealing is sometimes difficult. Moreover, when a fibrin sealant is used alone, there are problems that it is hard to press without a supporting material or that the strength is not satisfactory.

Accordingly, for ways and means of using a fibrin sealant, there have been various investigations and attempts for improvement. By way of example, in the field of respiratory surgery, a variety of devises have been done for prevention of an air leakage. Morikawa et al. has devised an approach in which a thrombin solution is applied to the pulmonary fistula and a strip of polyglactin mesh soaked with a fibrinogen solution is then attached thereto (see e.g. Non-patent reference 1). Although this approach has widely been adopted, it is still difficult to completely avoid recurrence of an air leakage. The cause of this insufficient effect of sealing is thought to be such that, since a thrombin solution is first applied, self fibrinogens present in the parenchyma of lung may form a thrombus to cover that site, which then prevents a fibrinogen solution of the adhesive preparation from penetrating to the interior of the tissue. In contrast to the approach according to Morikawa et al., another approach has been devised wherein a fibrinogen solution is first rubbed into the pulmonary fistula and then a fibrin sealant is applied thereto by spraying. Although this approach provided a high pressure resistance, as a fibrin sealant is used alone, it is not efficacious for emphysema due to insufficient strength and not being fixed to the afflicted site.

A fibrin sealant is used by dissolving each of the lyophilized fibrinogen and thrombin when used. Thus, taking time for dissolution, a fibrin sealant is not a dosage form suitable for use in an emergent operation or for a handy usage.

In order to eliminate preparing each of these ingredients of a fibrin sealant, there are attempts to fix the ingredients onto a variety of substrates to thereby produce a sheet type preparation. For such substrates, bioabsorbable/biodegradable materials have been used including natural components such as gelatin or collagen, or synthetic high molecular weight materials such as polyethylene glycol or polyglycolic acid. As an exemplary, a sheet preparation has been put into practice wherein horse-derived collagen holds fibrin and thrombin (e.g. Patent reference 1). However, the substrate collagen of this sheet preparation is rather thick and somewhat rigid to render the sheet preparation poorly stick to wounded regions where a sealing is desired, thereby making an efficacious sealing difficult. Besides, said sheet preparation is such that the substrate is made of equine collagen and thrombin is derived from bovine, i.e. material derived from non-human animal species is used, and hence, when it is for use in human, there is a possibility of induction of an antibody against heterologous proteins or onset of zoonotic infections such as prion disease, being far from ideal one.

In order to solve these problems, one approach is to develop a fibrin sealant that may ensure a thorough sealing in a short time. Such a fibrin sealant will be required to consist of the same coagulation factor as in human, when it is for use in human, free from infectious agents, to be in the form of a sheet so that a sealing effect may fully be exerted, and to use a sheet made of a material strictly selected and devised to be safe to the living body.
Patent reference 1: Japanese patent publication No. 34830/1986
Non-patent reference 1: Toshiaki Morikawa et al., 1994, The Japanese Association for Chest Surgery, Vol.8, p.288

### DISCLOSURE OF THE INVENTION

### (Technical Problem to be Solved by the Invention)

As described above, although various preparations and methods for a tissue sealing have been applied in the field of a variety of surgical operations, there has been no efficacious tissue sealant that has both efficacy and handiness.

### (Means for Solving the Problems)

In view of the above-mentioned various problems, the present inventors have carried out intensive investigation and as a consequence found that a tissue sealant comprising as an effective ingredient thrombin and fibrinogen characterized in that a bioabsorbable synthetic nonwoven fabric, which is a bioabsorbable synthetic material processed in the form of a nonwoven fabric, is used as a supporting material may exert quite excellent tissue sealing effects, to thereby complete the present invention.

Specifically, the present invention encompasses the following embodiments.
(1) A tissue sealant comprising as an effective ingredient thrombin and fibrinogen characterized in that a bioabsorbable synthetic nonwoven fabric is used as a supporting material;
(2) Use of a combination of a bioabsorbable synthetic nonwoven fabric as a supporting material and thrombin and fibrinogen as an effective ingredient for a tissue sealant;
(3) Use of a combination of a bioabsorbable synthetic nonwoven fabric holding thrombin as an effective ingredient, and fibrinogen as an effective ingredient for a tissue sealant;
(4) A tissue sealing kit comprising a bioabsorbable synthetic nonwoven fabric holding thrombin as an effective ingredient, and a container comprising fibrinogen as an effective ingredient; and
(5) A tissue sealing kit comprising a bioabsorbable synthetic nonwoven fabric as a substrate, a container comprising thrombin as an effective ingredient and a container comprising fibrinogen as an effective ingredient.

### (Effects of the Invention)

It was revealed that a tissue sealant according the present invention has the following properties and hence may be an ideal tissue sealant.
· It has an excellent sealing effect;
· It may be applied by its own sticky property and hence suture may be simplified or even made unnecessary;
· It may repair an afflicted site in a plane even at a wide range while retaining its shape;
· No trouble is required for dissolution and application is simple;
· It is excellent in safety;
· It does not cause adhesion which clinically matters;
· It is absorbed with time;
· It is excellent in flexibility and elasticity; and
· It causes merely a slight inflammatory reaction.
In accordance with the present invention, it is now possible to provide for a tissue sealant comprising a bioabsorbable synthetic nonwoven fabric which enables a safe, prompt and thorough tissue sealing in various clinical fields, typically in a surgical operation in various fields of the operation.

### BRIEF DESCRIPTION OF DRAWINGS

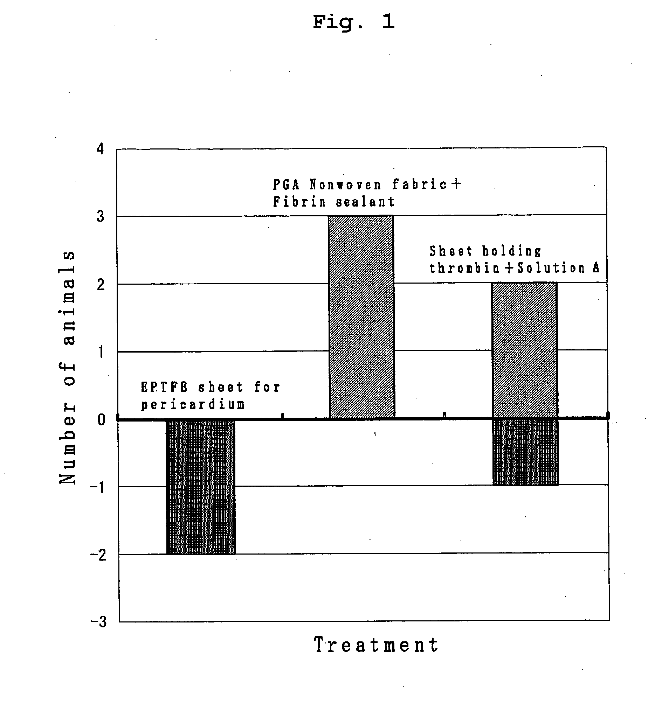

Fig. 1 is a figure showing reactions of the epicardium after a month from a treatment in a test evaluating a sealing effect using a canine pericardium defect model. In Fig. 1, positive values (oblique lines) depict a clinically admissible extent (slight or less) whereas negative values (lattice) depict a clinically undesirable extent (moderate or more).

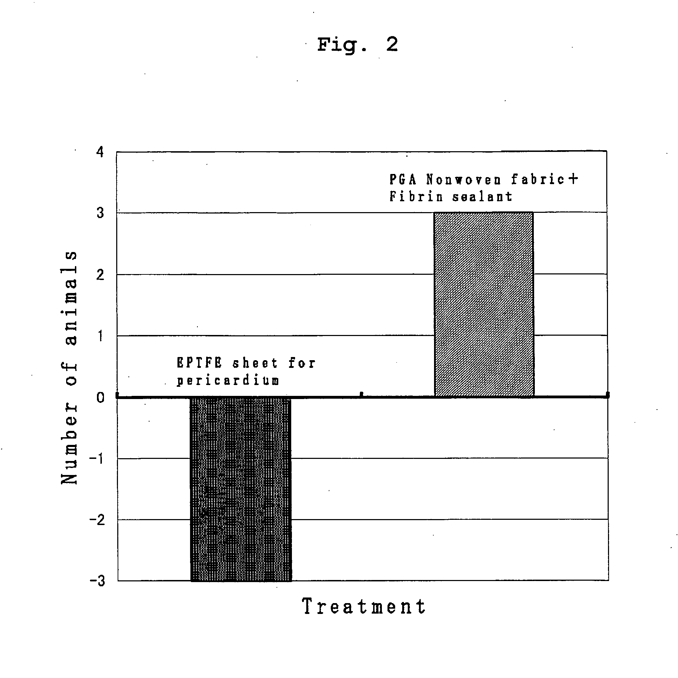

Fig. 2 is a figure showing reactions of the epicardium after two months from a treatment in a test evaluating a sealing effect using a canine pericardium defect model. In Fig. 2, positive values (oblique lines) depict a clinically admissible extent (slight or less) whereas negative values (lattice) depict a clinically undesirable extent (moderate or more).

### BEST MODE FOR CARRYING OUT THE INVENTION

The bioabsorbable synthetic nonwoven fabric for use in the present invention may be any nonwoven fabric made of a bioabsorbable synthetic fiber. A bioabsorbable synthetic fiber as used herein refers to a synthetic fiber that is unlikely to induce inflammation in the living body as a foreign substance and may be absorbed and/or degraded within the living body with time. The nonwoven fabric has preferably appropriate flexibility and elasticity to ensure that it may surely be stuck to any affected area. For example, a synthetic fiber that may form such a nonwoven fabric includes polyglycolic acid, polylactic acid, or a copolymer of glycolic acid with lactic acid, etc., which may be used after processing into a nonwoven fabric. Among these, a bioabsorbable synthetic nonwoven fabric which is prepared from polyglycolic acid by processing into a nonwoven fabric is the most preferable material for the purpose of the present invention.

The nonwoven fabric may be in any shape but preferably in the form of a sheet in view of versatility to various applications.

In addition to the effective ingredients, a pharmaceutically acceptable stabilizer and additive may also be added. Examples of such stabilizer and additive include, for instance, Factor XIII preferably derived from human blood or obtained by the genetic engineering, calcium chloride, a protease inhibitor (e.g. aprotinin), albumin, aminoacetic acid, polyethylene glycol, arginine, sodium hyaluronate, glycerol, mannitol, and the like.

Thrombin, fibrinogen and Factor XIII may preferably be derived from human blood or obtained by the genetic engineering. The tissue sealant of the present invention may be in any dosage form so far as thrombin and fibrinogen as an effective ingredient are ultimately contained in a bioabsorbable synthetic nonwoven fabric. In view of easy handling under operative settings, however, a bioabsorbable synthetic nonwoven fabric previously holding thrombin, which maintains flexibility, is one of preferable embodiments from the viewpoint of its easy handling as well as tissue sealing efficacy.

In case that a bioabsorbable synthetic nonwoven fabric previously holds both thrombin and fibrinogen, the nonwoven fabric should hold each of thrombin and fibrinogen under such condition that the components are separated from each other or each of the components in the form of powder are suspended in an organic solvent and each suspension is sprayed to the nonwoven fabric, so that both thrombin and fibrinogen may not react to each other to generate stabilized fibrin.

The tissue sealant of the present invention may be formulated as a kit comprising either:
(A) a bioabsorbable synthetic nonwoven fabric holding thrombin plus fibrinogen; or
(B) a bioabsorbable synthetic nonwoven fabric, thrombin, and fibrinogen;
in which a stabilizer and an additive as described above may optionally be added to both (A) and (B).

For use in case of (A), after fibrinogen is applied to an afflicted site, a bioabsorbable synthetic nonwoven fabric holding thrombin is overlaid, or alternatively, fibrinogen is applied to a bioabsorbable synthetic nonwoven fabric holding thrombin by spraying or by immersing. Said bioabsorbable synthetic nonwoven fabric holding thrombin may be prepared by (1) dissolving thrombin in a saline or a buffer and optionally adding to the resulting thrombin solution calcium chloride as an additive, and (2) immersing a bioabsorbable synthetic nonwoven fabric into said thrombin solution, followed by freezing at -80°C for 2 hours and lyophilization.

For use in case of (B), after fibrinogen, prepared as in the process for preparing a commercially available fibrin sealant (e.g. Bolheal manufactured by Juridical Foundation The Chemo-Sero-Therapeutic Research Institute), is applied to an afflicted site, a bioabsorbable synthetic nonwoven fabric immersed into the solution of thrombin is applied, or alternatively, each of the solutions of thrombin and fibrinogen is applied simultaneously to the nonwoven fabric via spray.

In either case of (A) or (B), Factor XIII or a protease inhibitor may be added to a solution containing fibrinogen.

The tissue sealant obtained in accordance with the present invention, due to its high adhesiveness, appropriate strength, flexibility and elasticity, may be stuck to a defect site of membranous tissues within the living body, a defect site or an incised surface of the organs and tissues or junction in any shape. Moreover, the tissue sealant of the present invention has a good biocompatibility and its own sticky property allows for a tissue sealing with simplified or no suture. Polyglycolic acid bioabsorbable nonwoven fabric as used for the substrate in the sealing material of the present invention, which has already been used for clinical purpose, is highly safe since it is absorbed within the living body and degraded into water and carbon dioxide.

As such, the tissue sealant according to the present invention may easily and quickly be applied to a defect site of membranous tissues within the living body, a defect site or an incised surface of the organs and tissues or junction and allow for an efficient tissue sealing through a blood coagulation reaction. Besides, since every material used therein is safe to the living body, it may be used in clinical without care.

### EXAMPLE

The present invention is explained in more detail by means of the following Examples but should not be construed to be limited thereto.

### Example 1: Preparation of sheet holding thrombin

A sheet holding thrombin in accordance with the present invention was prepared by the process as described below.

To a solution containing 5% human serum albumin are added 40 mM calcium chloride and subsequently thrombin at a final concentration of 500 U/mL. The solution is poured into a vessel at a depth of 1 mm where a bioabsorbable synthetic nonwoven fabric made of polyglycolic acid (Neoveil, Gunze Limited, thickness 0.15 mm) is laid on the bottom. The sheet, after being frozen at -80°C for 2 hours and lyophilized, is used as a sample of a sheet holding thrombin (thrombin held at 50 U/cm²).

Thrombin for use in this Example was prepared by the genetic engineering (cf. WO03/004641). Briefly, animal cells wherein a human prethrombin gene was incorporated were cultured and prethrombin was purified from its culture. On the other hand, ecarin purified from a culture of animal cells wherein an ecarin gene was incorporated was used for activation of prethrombin to thereby allow for purification of thrombin.

### Example 2: Test for evaluating sealing effect in canine pericardium defect model

This Example was performed by the procedures 1 to 7 as described below.
1. Beagles are anesthetized with xylazine and Ketalar.
2. Endotracheal intubation is done and the tube is connected to a respirator.
3. Intercostal thoracotomy at the left side is done.
4. The pericardium is excised (12mm×12mm) .
5. The defect site of the pericardium is treated with either of the sheets from the Groups as described below.
   Group 1: EPTFE sheet for pericardium
      An EPTFE sheet for pericardium (Gore-tex EPTFE Patch II (sheet for pericardium) /JGI, 15mmx15mm) is sutured to the pericardium with an EPTFE thread (Gore-tex Suture/JGI) by a single interrupted suture.
   Group 2: Polyglycolic acid nonwoven fabric + fibrin sealant
      To both surfaces of Neoveil (15mm×15mm) in a range of 12mm×12mm (the outside margin of 3mm is left for pasting-up) is sprayed a fibrin sealant (Bolheal, Juridical Foundation The Chemo-Sero-Therapeutic Research Institute, a solution containing fibrinogen and a solution containing thrombin; each about 0.25 mL/surface) to prepare a sheet. To the circumference of the defect site of the pericardium is dropped a fibrinogen solution (about 0.2 mL) which is rubbed thereto with the fingertip. Thereto is attached the above sheet and is sprayed a fibrin sealant (a solution containing fibrinogen and a solution containing thrombin; each about 0.5 mL).
   Group 3: Sheet holding thrombin + fibrinogen solution
      To the sheet holding thrombin prepared in Example 1 (15mm×15mm) in a range of 12mm×12mm (the outside margin of 3mm is left for pasting-up) is sprayed a fibrinogen solution (contained in Bolheal; about 0.5 mL/surface) to prepare a sheet. To the circumference of the defect site of the pericardium is dropped a fibrinogen solution (about 0.2 mL) which is rubbed thereto with the fingertip. Thereto is attached the above sheet and is sprayed a fibrinogen solution.
6. The test animals from all the Groups after a month from the treatment and from the Groups 1 and 2 after two months from the treatment are sacrificed and evaluated with the naked eye for an extent of adhesion and the reaction of the epicardium (whitening).
7. When adhesion is also observed in the tissues such as the pericardium, the cardiac muscle, etc. at the circumference of the sheet-attached site, said tissue may also be subject to histopathological examination.

As a result, both after a month and two months from the treatment, neither a peeling-off from the pericardium nor a damage of the treated sheet were observed in any of the Groups. For an extent of adhesion, its occurrence could be inhibited in Groups 2 and 3, corresponding to the present invention, in an extent equivalent to or even superior to that of Group 1 which has hitherto been used in clinical as shown in Table 1. As for the reaction of the epicardium, it was slighter in Groups 2 and 3 than in Group 1 as indicated in Fig. 1. In a histopathological test, inflammation in the epicardium could be seen in either Group. However, as shown in Fig. 2, the inflammation in Group 2 corresponding to the present invention tended to indicate remission after two months whereas hyperplasia of the connective tissue in the epicardium was further progressed and was more intense in Group 1 of the conventional method than in Group 2. For the pericardium, a layer of the connective tissue was formed in the surroundings of the sheet and mesothelial cells covered over said layer in Group 1. In Groups 2 and 3, as early as after a month from the treatment, hyperplasia of the connective tissue and vascularization could be seen not only in the surroundings but also in the interior of the sheet and mesothelial cells covered over the hyperplasia. It was thus revealed that the use of the tissue sealant according to the present invention allowed for rapid replacement with the living tissues and regeneration of the pericardium.

**Table 1**

| [After a month from the treatment] | | | | | | | | |
|---|---|---|---|---|---|---|---|---|
| Group | Treatment | Evaluation (extent) ¹⁾ | Adhesion site ²⁾ | | Total case number in each evaluation | Evaluation × Total case number | Evaluation × Total case number in total | Adhe sion index³⁾ |
| | | | Lung-Pericardium | Heart-Pericardium | | | | |
| 1 | EPTFE sheet for pericardium (n=2) | 0(-) | 0 | 2 | 2 | 0 | 6 | 3.0 |
| | | 1(±) | 0 | 0 | 0 | 0 | | |
| | | 2(+) | 0 | 0 | 0 | 0 | | |
| | | 3 (++) | 2 | 0 | 2 | 6 | | |
| | | 4 (+++) | 0 | 0 | 0 | 0 | | |
| 2 | PGA nonwoven fabric + fibrin sealant (n=3) | 0(-) | 1 | 1 | 2 | 0 | 10 | 3.3 |
| | | 1(±) | 0 | 0 | 0 | 0 | | |
| | | 2(+) | 0 | 2 | 2 | 4 | | |
| | | 3 (++) | 2 | 0 | 2 | 6 | | |
| | | 4 (+++) | 0 | 0 | 0 | 0 | | |
| 3 | Sheet holding thrombin + fibrinog en solution (n=3) | 0(-) | 2 | 0 | 4 | 0 | 6 | 2.0 |
| | | 1(±) | 0 | 0 | 0 | 0 | | |
| | | 2(+) | 0 | 0 | 0 | 0 | | |
| | | 3(++) | 1 | 1 | 2 | 6 | | |
| | | 4 (+++) | 0 | 0 | 0 | 0 | | |

| [After two months from the treatment] | | | | | | | | |
|---|---|---|---|---|---|---|---|---|
| Group | Treatment | Evaluation (extent) | Adhesion site | | Total case number in each evaluation | Evaluation × Total case number | Evaluation × Total case number in total | Adhe sion index |
| | | | Lung-Pericardiu | Heart-Pericardium | | | | |
| 1 | EPTFE sheet for pericard ium (n=3) | 0 (-) | 0 | 3 | 3 | 0 | 8 | 2.7 |
| | | 1(±) | 0 | 0 | 0 | 0 | | |
| | | 2(+) | 1 | 0 | 1 | 2 | | |
| | | 3(++) | 2 | 0 | 2 | 6 | | |
| | | 4(+++) | 0 | 0 | 0 | 0 | | |
| 2 | PGA nonwoven fabric + fibrin sealant (n=3) | 0(-) | 2 | 1 | 3 | 0 | 4 | 1.3 |
| | | 1(±) | 1 | 1 | 2 | 2 | | |
| | | 2(+) | 0 | 1 | 1 | 2 | | |
| | | 3 (++) | 0 | 0 | 0 | 0 | | |
| | | 4 (+++) | 0 | 0 | 0 | 0 | | |

| | | | | | | | | |
|---|---|---|---|---|---|---|---|---|
| 1) Criteria for evaluating an extent of adhesion and evaluation 0: -; No adhesion 1: ±; Slight adhesion (easily broken with fingertip) 2: +; Mild adhesion (broken by blunt dissection) 3: ++; Moderate adhesion (requiring sharp dissection) 4: +++; Severe adhesion (requiring sharp dissection in a wide range) 2) A case number in each of extents of adhesion for the respective adhesion sites 3) (Evaluation × Total case number in total)/number of animal treated | | | | | | | | |

### Example 3: Test for evaluating sealing effect in canine pulmonary air leakage model

This Example was performed by the procedures 1 to 7 as described below.
1. Beagles are anesthetized with Nembutal, endotracheal intubation is done and the tube is connected to a respirator.
2. Intercostal thoracotomy at the right side is done.
3. The lung is swelled and stretched and under natural deaeration carved edgewise along a paper pattern of 5mmx20mm with a knife.
4. The pleura of the wounded portion as carved edgewise is peeled off with a knife and bleeding, if any, is stopped with a cautery knife.
5. The air leakage is treated with either of the sheets from the Groups as described below.
   Group 1: Fibrinogen solution + sheet holding thrombin
      An 8% fibrinogen solution (about 0.7 mL) is applied to the air leakage site. The sheet holding thrombin prepared in Example 1 (10mm×25mm) is overlaid and an 8% fibrinogen solution is dropped thereto and the sheet is left to stand for 5 minutes.
   Group 2: Fibrinogen solution + thrombin solution + polyglycolic acid nonwoven fabric
      An 8% fibrinogen solution is applied to the air leakage site. Neoveil (10mm×25mm) used as a supporting material in Example 1 is overlaid and 0.7 mL of a solution containing thrombin (250 U/mL) as included in Bolheal is dropped thereto. Then, each about 0.7 mL of a solution containing fibrinogen and a solution containing thrombin as included in Bolheal is applied by spray and the sheet is left to stand for 5 minutes.
   Group 3: Collagen sheet preparation
      A collagen sheet preparation in which components of a fibrin sealant are fixed (TachoComb, Torii Pharmaceutical Co., Ltd.; fibrinogen and thrombin components are fixed by lyophilization on one surface of a sponge sheet made of equine collagen as a supporting material: 20mmx30mm), immersed in a saline, is attached to the air leakage site and left to stand for 5 minutes.
6. After treatment, the pressure is applied with a respirator while applying a saline to the air leakage site. A pressure when an air leakage is again induced is measured as a pressure resistance.
7. After measurement, the bronchia is nipped with a forceps and the treatment is repeated in different lobes. The measurement is done for three lobes, i.e. anterior, middle and lower lobes of either right or left lung.

As a result, Groups 1 and 2 wherein a polyglycolic acid nonwoven fabric is used as a supporting material exhibited higher pressure resistance efficiency than that of Group 3 wherein a collagen sheet preparation is used. In particular, Group 1 wherein a sheet holding thrombin is used exhibited especially higher pressure resistance efficiency. In general, after a surgical operation in the respiratory system, a patient is often bade to cough for the purpose of prevention from infection wherein an instantaneous inner pressure of the interior of the airway is reportedly 40 to 50 cm · H₂O. Group 3 wherein a collagen sheet preparation is used exhibited a mean pressure resistance of 34.6 cm H₂O and hence is not expected to provide a sufficient sealing efficacy. The method according to the present invention allows for a tissue sealing which is simpler and more effective than the conventional methods used in clinical.

**Table 2**

| Treatment | Mean pressure resistance/cm · H₂O (n=5) |
|---|---|
| Group 1: Fibrinogen + sheet holding thrombin | 54±6. 28 54±6.28 |
| Group 2: Fibrinogen + thrombin + PGA nonwoven fabric | 42.4±7.47 |
| Group 3: Collagen sheet preparation | 34.6±9.69 |

### INDUSTRIAL APPLICABILITY

The tissue sealant according to the present invention has a good biocompatibility and its own sticky property allows for a tissue sealing with simplified or no suture. Accordingly, it may be used for sealing a defect site of membranous tissues within the living body such as the pleura, the pericardium or the serosa, a defect site or an incised surface of the organs and tissues or junction.

## Claims

1. A tissue sealant comprising as an effective ingredient thrombin and fibrinogen **characterized in that** a bioabsorbable synthetic nonwoven fabric is used as a supporting material.

2. The tissue sealant according to claim 1, wherein said bioabsorbable synthetic nonwoven fabric is made of a material selected from the group consisting of polyglycolic acid, polylactic acid and a copolymer of glycolic acid and lactic acid.

3. The tissue sealant according to claim 2, wherein said bioabsorbable synthetic nonwoven fabric is a nonwoven fabric made of a material of polyglycolic acid.

4. The tissue sealant according to any one of claims 1 to 3, wherein the bioabsorbable synthetic nonwoven fabric previously holds at least thrombin among thrombin and fibrinogen.

5. The tissue sealant according to any one of claims 1 to 4, wherein said tissue sealant comprises at least one additive selected from Factor XIII, a protease inhibitor, or calcium chloride.

6. The tissue sealant according to any one of claims 1 to 5, wherein thrombin, fibrinogen and Factor XIII are either derived from human blood or produced by a genetic engineering.

7. The tissue sealant according to any one of claims 1 to 6, wherein said tissue sealant is used for sealing a defect site or an incised surface of the organs and tissues, or for sealing junction between incised tissues or between incised tissues and prosthetic materials.

8. Use of a combination of a bioabsorbable synthetic nonwoven fabric as a supporting material and thrombin and fibrinogen as an effective ingredient for a tissue sealant.

9. The use according to claim 8, wherein said bioabsorbable synthetic nonwoven fabric is made of a material selected from the group consisting of polyglycolic acid, polylactic acid and a copolymer of glycolic acid and lactic acid.

10. The use according to claim 8 or 9, wherein said bioabsorbable synthetic nonwoven fabric is a nonwoven fabric made of a material of polyglycolic acid.

11. The use according to any one of claims 8 to 10,
wherein said tissue sealant comprises at least one additive selected from Factor XIII, a protease inhibitor, or calcium chloride.

12. The use according to any one of claims 8 to 11,
wherein said Factor XIII is added to fibrinogen.

13. The use according to any one of claims 8 to 12,
wherein said thrombin, fibrinogen and Factor XIII are either derived from human blood or produced by a genetic engineering.

14. The use according to any one of claims 8 to 13,
wherein said tissue sealant is used for sealing a defect site or an incised surface of the organs and tissues, or for sealing junction between incised tissues or between incised tissues and prosthetic materials.

15. Use of a combination of a bioabsorbable synthetic nonwoven fabric holding thrombin as an effective ingredient, and fibrinogen as an effective ingredient for a tissue sealant.

16. The use according to claim 15, wherein said bioabsorbable synthetic nonwoven fabric holding thrombin as an effective ingredient is prepared by the steps of immersing a bioabsorbable synthetic nonwoven fabric into a solution containing thrombin and of lyophilizing the obtained nonwoven fabric.

17. The use according to claim 15 or 16, wherein said bioabsorbable synthetic nonwoven fabric is made of a material selected from the group consisting of polyglycolic acid, polylactic acid and a copolymer of glycolic acid and lactic acid.

18. The use according to claim 17, wherein said bioabsorbable synthetic nonwoven fabric is a nonwoven fabric made of a material of polyglycolic acid.

19. The use according to any one of claims 15 to 18,
wherein said tissue sealant comprises at least one additive selected from Factor XIII, a protease inhibitor, or calcium chloride.

20. The use according to claim 19, wherein said calcium chloride is fixed to the bioabsorbable synthetic nonwoven fabric together with thrombin.

21. The use according to claim 19, wherein said Factor XIII is added to fibrinogen.

22. The use according to any one of claims 15 to 21,
wherein said thrombin, fibrinogen and Factor XIII are either derived from human blood or produced by a genetic engineering.

23. The use according to any one of claims 15 to 22,
wherein said tissue sealant is used for sealing a defect site or an incised surface of the organs and tissues, or for sealing junction between incised tissues or between incised tissues and prosthetic materials.

24. A tissue sealing kit comprising a bioabsorbable synthetic nonwoven fabric holding thrombin as an effective ingredient, and a container comprising fibrinogen as an effective ingredient.

25. The tissue sealing kit according to claim 24,
wherein said bioabsorbable synthetic nonwoven fabric is made of a material selected from the group consisting of polyglycolic acid, polylactic acid and a copolymer of glycolic acid and lactic acid.

26. The tissue sealing kit according to claim 25,
wherein said bioabsorbable synthetic nonwoven fabric is a nonwoven fabric made of a material of polyglycolic acid.

27. The tissue sealing kit according to any one of claims 24 to 26, wherein said tissue sealing kit comprises at least one additive selected from Factor XIII, a protease inhibitor, or calcium chloride.

28. The tissue sealing kit according to claim 27,
wherein said calcium chloride is added to the bioabsorbable synthetic nonwoven fabric as an additive for thrombin.

29. The tissue sealing kit according to claim 27,
wherein said Factor XIII is included in a container comprising fibrinogen.

30. The tissue sealing kit according to any one of claims 24 to 29, wherein said thrombin, fibrinogen and Factor XIII are either derived from human blood or produced by a genetic engineering.

31. The tissue sealing kit according to any one of claims 24 to 30, wherein said bioabsorbable synthetic nonwoven fabric holding thrombin is prepared by the steps of immersing a bioabsorbable synthetic nonwoven fabric into a solution containing thrombin and of lyophilizing the obtained nonwoven fabric.

32. The tissue sealing kit according to any one of claims 24 to 31, wherein said tissue sealing kit is used for sealing a defect site or an incised surface of the organs and tissues, or for sealing junction between incised tissues or between incised tissues and prosthetic materials.

33. A tissue sealing kit comprising a bioabsorbable synthetic nonwoven fabric as a substrate, a container comprising thrombin as an effective ingredient and a container comprising fibrinogen as an effective ingredient.

34. The tissue sealing kit according to claim 33,
wherein said bioabsorbable synthetic nonwoven fabric is made of a material selected from the group consisting of polyglycolic acid, polylactic acid and a copolymer of glycolic acid and lactic acid.

35. The tissue sealing kit according to claim 34,
wherein said bioabsorbable synthetic nonwoven fabric is a nonwoven fabric made of a material of polyglycolic acid.

36. The tissue sealing kit according to any one of claims 33 to 35, wherein said tissue sealing kit comprises at least one additive selected from Factor XIII, a protease inhibitor, or calcium chloride.

37. The tissue sealing kit according to claim 36,
wherein said Factor XIII is included in a container comprising fibrinogen.

38. The tissue sealing kit according to any one of claims 33 to 37, wherein said thrombin, fibrinogen and Factor XIII are either derived from human blood or produced by a genetic engineering.

39. The tissue sealing kit according to any one of claims 33 to 38, wherein said tissue sealing kit is used for sealing a defect site or an incised surface of the organs and tissues, or for sealing junction between incised tissues or between incised tissues and prosthetic materials.
